# EUROPÄISCHE PATENTSCHRIFT

(11) **EP 4 609 890 B1**
(45) Veröffentlichungstag und Bekanntmachung des Hinweises auf die Patenterteilung: **28.10.2026**
(21) Anmeldenummer: 24161013.8
(22) Anmeldetag: 01.03.2024
(51) Int. Cl.: A61M 1/16

(54) **BEHÄLTNIS ZUR AUFNAHME EINES FLÜSSIGEN DIALYSEKONZENTRATS UND DIALYSEKONZENTRAT-EINSPEISEVORRICHTUNG ZUR BEREITSTELLUNG VON DIALYSEKONZENTRAT**
CONTAINER FOR RECEIVING A LIQUID DIALYSIS CONCENTRATE AND DIALYSIS CONCENTRATE SUPPLY DEVICE FOR PROVIDING DIALYSIS CONCENTRATE
RÉCIPIENT DESTINÉ À RECEVOIR UN CONCENTRÉ DE DIALYSE LIQUIDE ET DISPOSITIF D'ALIMENTATION DE CONCENTRÉ DE DIALYSE POUR FOURNIR UN CONCENTRÉ DE DIALYSE

(43) Veröffentlichungstag der Anmeldung: 03.09.2025
(73) Patentinhaber: Fresenius Medical Care Deutschland GmbH, 61352 Bad Homburg (DE)
(72) Erfinder: RABAHI, Myriam, 61352 Bad Homburg (DE); HEMM, Andreas, 61352 Bad Homburg (DE); SCHEEL, Andreas, 61352 Bad Homburg (DE); DOUCET, Matthieu, 61352 Bad Homburg (DE); MAI, Michael, 61352 Bad Homburg (DE); LAFFAY, Philippe, 61352 Bad Homburg (DE)
(74) Vertreter: Oppermann, Frank

(56) Entgegenhaltungen:
- US-A- 5 794 669
- US-A1- 2003 034 305

## Beschreibung

Die Erfindung betrifft ein Behältnis zum Anschluss an eine Schlauchleitung einer Dialysekonzentrat-Einspeisevorrichtung zur Bereitstellung von Dialysekonzentrat für die Herstellung einer gebrauchsfertigen Dialysierflüssigkeit in einem Dialysezentrum oder Krankenhaus, ein Gebinde aufweisend einen Faltkarton und ein derartiges Behältnis sowie ein Verfahren zur Herstellung eines derartigen Behältnisses. Darüber hinaus betrifft die Erfindung eine Dialysekonzentrat-Einspeisevorrichtung zur Bereitstellung von Dialysekonzentrat für die Herstellung einer gebrauchsfertigen Dialysierflüssigkeit.

Zur Herstellung von Dialysierflüssigkeit, auch Dialysat genannt, können vorgefertigte Dialysekonzentrate verwendet werden, die mit einer vorgegebenen Menge an Permeat (Reinwasser, das im Kontext der Dialyse auch als Dialysewasser bezeichnet wird) verdünnt werden, um eine gebrauchsfertige Dialysierflüssigkeit zu erhalten. Als Konzentrate werden saure Konzentrate, insbesondere Acetatkonzentrate, oder basische Konzentrate, insbesondere Bicarbonatkonzentrate, verwendet. Permeat und Konzentrat werden in einem sogenannten Mischkreis der Dialysevorrichtung gemischt.

Um die einzelnen Dialysevorrichtungen in einem Dialysezentrum oder Krankenhaus mit Dialysekonzentrat versorgen zu können, kann in dem Dialysezentrum oder Krankenhaus ein Leitungssystem vorgesehen sein, an welches die einzelnen Dialysemaschinen angeschlossen werden. Dieses Leitungssystem kann eine oder mehrere Ringleitungen und/oder Stichleitungen umfassen. Die Einspeisung des Dialysekonzentrats in die Ringleitung erfolgt mit einer Dialysekonzentrat-Einspeisevorrichtung. US5794669 beschreibt ein Behältnis zum Anschluss an eine Dialysemaschine zur Bereitstellung von Dialysekonzentrat für die Herstellung einer gebrauchsfertigen Dialysierflüssigkeit in einem Dialysezentrum oder Krankenhaus.

Das Dialysekonzentrat wird zur Einspeisung mit der Dialysekonzentrat-Einspeisevorrichtung in Behältnissen bereitgestellt, welche eine größere Menge an Dialysekonzentrat, beispielsweise 300 I, aufnehmen können.

Die Dialysekonzentrat-Einspeisevorrichtung weist eine Schlauchleitung auf, an deren freiem Ende ein schlauchseitiges Anschlussstück vorgesehen ist, welches mit dem beutelseitigen Anschlussstück des Behältnisses verbunden wird. Eine Dialysekonzentrat-Einspeisevorrichtung kann aus Gründen der Redundanz auch über mehrere Schlauchleitungen verfügen, um mehrere Behältnisse anschließen zu können.

An die Handhabung der zur einmaligen Verwendung bestimmten Behältnisse werden in der Praxis große Anforderungen gestellt. Die Behältnisse sollten sich leicht transportieren und an die Dialysekonzentrat-Einspeisevorrichtung anschließen lassen, ohne dass die Gefahr von Leckagen besteht. Darüber hinaus sollten sich die beutelseitigen Anschlusstücke der Behältnisse mit einem möglichst geringen fertigungstechnischen Aufwand herstellen lassen und die Behältnisse sollten sich leicht entsorgen lassen.

Der Erfindung liegt die Aufgabe zugrunde, ein Behältnis zum Anschluss an eine Schlauchleitung einer Dialysekonzentrat-Einspeisevorrichtung zur Bereitstellung von Dialysekonzentrat für die Herstellung einer gebrauchsfertigen Dialysierflüssigkeit in einem Dialysezentrum oder Krankenhaus bereitzustellen, welches den oben genannten Anforderungen genügt. Der Erfindung liegt auch die Aufgabe zugrunde, ein Verfahren anzugeben, mit dem sich ein derartiges Behältnis mit einem verhältnismäßig geringen fertigungstechnischen Aufwand kostengünstig herstellen lässt. Eine Aufgabe der Erfindung ist auch, eine Dialysekonzentrat-Einspeisevorrichtung und ein Dialysekonzentrat-Verteilungssystem für ein derartiges Behältnis bereitzustellen.

Die Lösung dieser Aufgaben erfolgt erfindungsgemäß mit den Merkmalen der unabhängigen Patentansprüche. Die abhängigen Ansprüche betreffen vorteilhafte Ausführungsformen der Erfindung.

Die nachfolgend beschriebenen Ausführungsformen der Erfindung können eines oder mehrere der nachfolgend genannten Merkmale oder Merkmalskombinationen umfassen. Ein mit einem unbestimmten Artikel bezeichnetes Merkmal kann auch mehrfach vorhanden sein, wenn der unbestimmte Artikel nicht mit einem ausdrücklichen Hinweis auf eine nur einmalige Verwendung zu verstehen ist. Eine Bezeichnung von Merkmalen mit einem Zahlwort, beispielsweise "erstes und zweites", schließt nicht aus, dass über die durch das Zahlwort angegebene Anzahl hinaus diese Merkmale noch weitere Male vorhanden sein können. Bei der Beschreibung sämtlicher Ausführungsformen ist der Ausdruck "kann" auch als "vorzugsweise" oder "zweckmäßigerweise" zu verstehen.

Der Folienbeutel des erfindungsgemäßen Behältnisses, welcher ein vorgegebenes Innenvolumen zur Aufnahme eines flüssigen Dialysekonzentrats umschließt, zeichnet sich durch eine in der Gebrauchslage untere Seite und eine obere Seite aus. Dabei ist unter Gebrauchslage des Folienbeutels die Lage zu verstehen, in welcher der Beutel an die Dialysekonzentrat-Einspeisevorrichtung angeschlossen ist und auf dem Boden aufsteht. Ansonsten kann der Beutel grundsätzlich eine beliebige Form haben. Der Folienbeutel kann ein Flachbeutel oder Standbodenbeutel oder ein Beutel sein, welcher sich beim Befüllen entfalten und eine Form annehmen kann, in der sich der Beutel passend in einen Karton mit flachen Außenwänden einsetzten lässt.

Die Erfindung betrifft insbesondere, ist aber nicht darauf beschränkt, ein Großbehältnis, d.h. ein Behältnis mit einem Innenvolumen von größer als 50 I, vorzugsweise größer als 200 I, beispielsweise 300 I, so dass das Behältnis eine Menge an Dialysekonzentrat aufnehmen kann, das für eine Vielzahl von Behandlungen ausreichend ist. Das Innenvolumen eines derartigen Behältnisses unterscheidet das Behältnis von denjenigen kleineren Behältnissen, die ein Innenvolumen haben, das nur eine Menge an Dialysekonzentrat für nur eine Behandlung aufnehmen können.

An der Oberseite des Folienbeutels ist ein beutelseitiges Anschlussstück vorgesehen, welches zum Anschluss eines schlauchleitungsseitigen, komplementären Anschlussstücks einer Schlauchleitung ausgebildet ist. Das beutelseitige Anschlussstück weist ein Basisteil auf, welches an der Oberseite des Folienbeutels mit den Folienlagen, beispielweise im Bereich einer Schweißnaht an der Oberseite des Beutels, flüssigkeitsdicht verbunden ist. In dem Basisteil ist ein vorzugsweise zentral angeordneter Durchgang vorgesehen. Das Basisteil kann die Form eines "Schiffchens" haben, das sich mit den Folienlagen des Beutels leicht verschweißen lässt.

Für die Handhabung des Behältnisses ist von Vorteil, dass sich das beutelseitige Anschlussstück in der Gebrauchslage des Beutels oben befindet, so dass das beutelseitige Anschlussstück für die Konnektion eines schlauchseitigen Anschlussstücks leicht zugänglich ist. Dadurch ist sichergestellt, dass nach der Diskonnektion des schlauchseitigen Anschlussstücks Dialysekonzentrat nicht auslaufen kann, wenn das Behältnis nicht vollständig entleert sein sollte. Ein weiterer Vorteil liegt in der verbesserten Ergonomie bei der Handhabung des Behältnisses. Die Konnektion an einem obenliegenden Anschluss ist nicht nur bequemer, sondern bei einem obenliegenden Anschluss besteht im Gegensatz zu einem an der Seite des Behälters liegenden Anschluss auch ein geringeres Risiko einer Beschädigung durch Kollisionen insbesondere bei dem Transport des Behälters auf einem Wagen. Allerdings erfordert ein obenliegender Sauganschluss zusätzliche Maßnahmen, um Flüssigkeit und nicht etwa Luft entnehmen zu können.

Das beutelseitige Anschlussstück des erfindungsgemäßen Behältnisses zeichnet sich durch ein in den Durchgang des Basisteils eingesetztes Einsatzteil aus, welches einen vorzugsweise zentralen Durchlass für das Dialysekonzentrat aufweist, welcher von einem abnehmbaren oder abtrennbaren Verschlusselement flüssigkeitsdicht verschlossen werden kann bzw. sein kann. Ein Verschluss des Durchlasses ist zwar nicht zwingend erforderlich, bietet aber Vorteile insbesondere im Hinblick auf den Transport zu unterschiedlichen Fertigungsstätten. Das Verschlusselement ist vorteilhafterweise eine abziehbare Folie, die vorzugsweise auf das obere Endstück des Einsatzteils aufgebracht ist. Diese Folie verschließt das beutelseitige Anschlussstück flüssigkeitsdicht und kann leicht entfernt werden.

Das Einsatzteil des beutelseitigen Anschlussstücks, das aus einem Stück hergestellt (einstückig) ist, kann in ein Oberteil und ein Unterteil untergliedert werden. Das Oberteil des Einsatzteils ist in der Art eines männlichen Steckers einer sogenannten Camlock-Kupplung ausgebildet, welcher sich durch eine umlaufende Nut auszeichnet.

Eine Camlock-Kupplung als solche, die auch als Nuten- oder Nockenkupplung bezeichnet wird, gehört zum Stand der Technik und wird beispielsweise in der Öl- und Gasindustrie, der chemischen Industrie, im verarbeitenden Gewerbe oder der Landwirtschaft eingesetzt. Im Kontext von Behältnissen für flüssiges Dialysekonzentrat ist die Verwendung von Camlock-Kupplungen aber nicht bekannt.

Die bekannten Camlock-Kupplungen, die im Stand der Technik zum Verbinden von flüssigkeitsführenden Schlauchleitungen Verwendung finden, umfassen einen männlichen Stecker und ein weibliches Kupplungsstück. Zur Herstellung der Verbindung wird der männliche Stecker, der mit einer umlaufenden Nut versehen ist, in das weibliche Kupplungsstück eingesetzt. Das weibliche Kupplungsstück weist schwenkbar gelagerte Verriegelungsarme mit Nocken auf, die beim Umlegen der Verriegelungsarme in die umlaufende Nut des Steckers eingreifen.

Da das Oberteil des Einsatzteils des beutelseitigen Anschlussstücks in der Art eines männlichen Steckers einer Camlock-Kupplung ausgebildet ist, kann eine flüssigkeitsdichte Strömungsverbindung zwischen dem Behältnis und der Schlauchleitung sehr einfach geschaffen werden. Von Vorteil ist, dass zur Herstellung der Verbindung das beutelseitige Anschlussstück des zur einmaligen Verwendung bestimmten Behältnisses nicht über bewegliche Teile zu verfügen braucht, sondern sich nur durch eine umlaufende Nut auszeichnen muss, wodurch sich der Fertigungsaufwand verringert.

Die bekannten Cam-Lock-Kupplungen genügen einem bestimmten Standard, um kompatibel zu sein. Diesem Standard braucht das in der Art eines männlichen Steckers einer Camlock-Kupplung ausgebildete Oberteil des beutelseitigen Anschlussstücks aber nicht zu genügen. Wenn daher davon die Rede ist, dass Elemente der vorliegenden Kupplung in der Art eines männlichen Steckers bzw. eines weiblichen Kupplungsstücks einer Camlock-Kupplung ausgebildet sind, ist damit nur gemeint, dass die Elemente so gestaltet sind, dass sie das Prinzip der Camlock-Kupplung verwirklichen, die sich durch in eine Nut eines Steckers (männliches Kupplungsstück) eingreifende Verriegelungsarme einer Buchse (weibliches Kupplungsstück) auszeichnet. Die weitergehende Gestaltung, insbesondere auf der dem weiblichen Kupplungsstück abgewandten Seite, kann jedoch deutlich von den Spezifikationen, insbesondere den standardisierten Abmessungen, der bekannten Camlock-Kupplungen abweichen, was bereits den speziellen Anforderungen für Dialysekonzentrat geschuldet ist, beispielsweise hinsichtlich Dichtigkeit oder Druckbeständigkeit. In diesem Zusammenhang sei bemerkt, dass im Gegensatz zu der Wassertechnik bei der Versorgung einer Dialysemaschine mit Dialysekonzentrat höhere Anforderungen an die Dichtigkeit des Systems gestellt werden. Sollten bei dem Anschluss einer Wasserleitung geringfüge Undichtigkeiten auftreten, spielen diese in der Praxis keine große Rolle, da das austretende Wasser relativ schnell verdunstet. Bei der Versorgung einer Dialysemaschine mit Dialysekonzentrat können sich aber schon bei kleineren Leckagen Kristalle ausbilden, welche die Undichtigkeit sogar noch erhöhen können.

Im Gegensatz zu anderen Kupplungsarten, welche ggf. ohne Betätigung eines Hebels oder Druckknopfs dichtend einrasten, bietet die Camlock-Verbindungstechnik den Vorteil einer unmittelbaren mechanischen Rückmeldung, ob eine dichtende, korrekte Verbindung hergestellt wurde oder nicht. Denn aufgrund der besonderen Ausbildung der Nuten des Steckers und der Nocken der Verriegelungsarme der Buchse für eine klemmende Befestigung erlaubt ein Verschwenken der Verriegelungsarme nur bei korrekter Anordnung von Stecker und Buchse zueinander ein Erreichen der erwünschten Dichtwirkung.

Das Unterteil des Einsatzteils des beutelseitigen Anschlussstücks umfasst ein im Wesentlichen hohlzylindrisches äußeres Einsatzstück, welches bei der Fertigung des Anschlussstücks leicht in den Durchgang des Basisteil eingesetzt werden kann bzw. bei dem fertigen Anschlussstück eingesetzt ist, und ein im Wesentlichen hohlzylindrisches inneres Verbindungsstück, an welches sich ein in das Innenvolumen des Beutels erstreckender Schlauch leicht anschließen lässt bzw. angeschlossen ist. Der sich in das Innere vorzugsweise bis an den unteren Rand oder Boden des Folienbeutels erstreckende Schlauch erlaubt die Anordnung des beutelseitigen Anschlussstücks an der Beuteloberseite, wobei der Beutel vollständig entleert werden kann.

Ein weiterer Vorteil liegt darin, dass das beutelseitige Anschlussstück aus nur zwei Teilen bestehen kann, d.h. einem einstückigen Basisteil und einem einstückigen Einsatzteil, welche sich als Spritzgießteile im Spritzgießverfahren in großen Stückzahlen kostengünstig herstellen lassen. Basisteil und Einsatzteil erlauben eine einfache Befüllung des Behältnisses mit Dialysekonzentrat in einer Füllstation und den einfachen Verschluss des Behältnisses nach der Befüllung. Die Reduzierung der Anzahl der Teile verringert nicht nur die Fertigungskosten, sondern auch die Gefahr von Leckagen, da nur zwei Teile gegeneinander abgedichtet werden müssen.

Die Erfindung betrifft nicht nur das leere Behältnis, sondern auch ein Behältnis, das mit einem flüssigen Dialysekonzentrat, insbesondere mit einem Säurekonzentrat zur Herstellung einer gebrauchsfertigen Dialysierflüssigkeit Hämodialyse befüllt ist.

Eine Ausführungsform des Behältnisses sieht vor, dass das im Wesentlichen hohlzylindrische äußere Einsatzstück des Unterteils des Einsatzteils des beutelseitigen Anschlussstücks einrastend und/oder klemmend in den Durchgang des Basisteils des beutelseitigen Anschlussstücks eingepresst ist, um die beiden Teile flüssigkeitsdicht miteinander zu verbinden. Dadurch kann eine relativ aufwendige Schweißverbindung entfallen, welche für den Fertigungsprozess eine zusätzliche Schweißstation erfordert. Die einrastende und/oder klemmende Verbindung kann somit mit einem relativ einfachen in der Art einer Zange ausgebildeten Werkzeug unmittelbar nach dem Befüllen des Folienbeutels oder auch später von Hand erfolgen.

Die Camlock-Verbindungstechnik schafft bei dem beutelseitigen Anschlussstück eine relativ große Anlagefläche, an welcher sich eine abziehbare Folie zum Verschluss des Durchlasses in dem Einsatzteil sicher befestigen lässt. Des Weiteren verringert sich mit einem vergrößerten Einlass auch die Gefahr einer Kristallisation an dem oberen Endstück des Einsatzteils, da am oberen Rand anstehendes Dialysekonzentrat damit leichter in den Beutel zurückfließen kann.

Bei einer weiteren Ausführungsform ist das obere Endstück des Schlauchs in das im Wesentlichen hohlzylindrische innere Verbindungsstück des Unterteils des Einsatzteils eingeschoben. Bei einer entsprechenden Dimensionierung des Innendurchmessers des inneren Verbindungsstücks und des Außendurchmessers des Schlauchs kann somit auf eine relativ aufwendige Schweiß- oder Klebeverbindung verzichtet werden, um den Schlauch an dem Einsatzteil sicher zu befestigen. Das Verbindungsstück kann aber auch als eine Schlauchtülle zum Aufschieben der Schlauchleitung ausgebildet sein.

Das Basisteil des beutelseitigen Anschlussstücks weist vorzugsweise einen oberen umlaufenden Flansch auf, um das Basisteil an dem Deckelteil eines Faltkartons als Umverpackung abstützen zu können. Das Einsatzteil kann wiederum einen umlaufenden Rand aufweisen, welcher sich auf der Oberseite des umlaufenden Flansches des Basisteils abstützen kann.

Auf das Oberteil des Einsatzteils des beutelseitigen Anschlussstücks kann eine Verschluss- oder Schutzkappe aufgesetzt werden, um die obere Öffnung des Einsatzteils zu verschließen oder die bereits verschlossene Öffnung, insbesondere die abziehbare Folie, zu schützen. Auch für den Verschluss des Anschlussstücks mit einer Verschluss- oder Schutzkappe erweist sich die relativ große Anlagefläche des Einsatzteils als vorteilhaft.

Das mit Dialysekonzentrat befüllte Behältnis kann in einem Gebinde bereitgestellt werden, welches das Behältnis und einen Faltkarton als Umverpackung mit einem Bodenteil, Seitenteilen und einem ein- oder mehrteiligen Deckelteil umfasst. Das Behältnis ist in den Faltkarton derart eingesetzt, dass der Folienbeutel auf dem Bodenteil aufsteht, wobei sich das Oberteil des Einsatzteils durch den Deckelteil nach außen erstreckt. Der Faltkarton verleiht dem Beutel eine ausreichende Stabilität und Standfestigkeit und vereinfacht den Transport zu der Dialysekonzentrat-Einspeisevorrichtung. Die Gebinde können somit auf geeigneten Paletten aufgenommen und transportiert werden.

Die Erfindung betrifft auch eine Dialysekonzentrat-Einspeisevorrichtung, die einen einfachen und sicheren Anschluss des Behältnisses ermöglicht. Die Dialysekonzentrat-Einspeisevorrichtung verfügt über eine Schlauchleitung zum Ansaugen von Dialysekonzentrat, an deren Ende ein komplementäres Anschlussstück zur Herstellung einer Flüssigkeitsverbindung mit dem beutelseitigen Anschlussstück vorgesehen ist. Das schlauchseitige Anschlussstück ist in der Art eines weiblichen Kupplungsstücks der Camlock-Kupplung ausgebildet, welches zur Aufnahme eines in der Art eines männlichen Steckers der Camlock-Kupplung ausgebildeten Steckers ausgebildet ist und welches zwei Verriegelungsarme zur Verriegelung des männlichen Steckers in dem weiblichen Kupplungsstück aufweist. Das weibliche Kupplungsstück ist weiterhin als ein kappenförmiger Körper ausgebildet, welcher ein Basisteil, in dem eine Ausnehmung für den männlichen Stecker vorgesehen ist, und einen Deckelteil aufweist,
wobei der Deckelteil mit einer Anschlusstülle versehen ist, an welche das freie Ende der Schlauchleitung angeschlossen ist.

Die im Bereich der Wassertechnik bekannten Camlock-Kupplungen sind im Allgemeinen für den Anschluss von Schlauchleitungen mit einem relativ großen Durchmesser (z.B. 1 Zoll, 1 ½ Zoll) ausgelegt und verfügen über einen Gewindeanschluss, der sich für Schlauchleitungen mit einen relativ kleinen Durchmesser, die in dem medizintechnischen Bereich der Dialyse verwendet werden, selbst bei der Verwendung eines Dichtbandes im Hinblick auf die Dichtigkeit als problematisch erweist, weshalb eine Kristallisation stattfinden kann. Die Anschlusstülle der erfindungsgemäßen Camlock-Kupplung erlaubt hingegen den Anschluss von Schlauchleitungen mit einem relativ kleinen Durchmesser (z.B. 12 mm), welche aufgrund ihres kleinen Durchmessers an sich nur eine relativ kleine Dichtfläche zur Verfügung stellen, mittels einer konventionellen Schlauchklemme. Aufgrund des relativ großen Überlappungsbereichs von Schlauch und Tülle ist die Dichtfläche dennoch ausreichend groß, um eine Kristallisation wirkungsvoll zu verhindern.

Eine bevorzugte Ausführungsform sieht vor, dass die Längsachse der Anschlusstülle gegenüber der Längsachse der Ausnehmung vorzugsweise nach oben weisend schräggestellt ist. Der von der Längsachse der Anschlusstülle und der Längsachse der Ausnehmung eingeschlossene Winkel liegt vorzugsweise zwischen 60-75°, besonders bevorzugt 70°. Dadurch wird eine kompakte Bauform erzielt und ein unbeabsichtigtes Abknicken des Schlauches insbesondere bei der Verbindung von Stecker und Kupplungsstück verhindert.

Bei den bekannten Camlock-Kupplungen, die sich durch eine gerade Führung der miteinander verbundenen Schlauchleitungen auszeichnen, besteht die Gefahr des Abknickens der flüssigkeitsgefüllten Schlauchleitung aufgrund ihres Eigengewichts. In der Praxis hat sich gezeigt, dass diese Gefahr bei der erfindungsgemäßen Camlock-Kupplung wegen der schonenderen Führung des abgewinkelten Schlauchs geringer ist.

Eine weitere bevorzugte Ausführungsform sieht vor, dass in der Ausnehmung des Basisteils des schlauchseitigen Anschlussstücks eine sich gegenüber dem Deckelteil abstützende Dichtung aus einem Vollmaterial mit einem im Wesentlichen rechteckförmigen Querschnitt, beispielsweise eine Ring- oder Flachdichtung, angeordnet ist, welche im Gegensatz zu bei Camlock-Verbindungen verwendeten Lippendichtungen eine relativ große Anlage- bzw. Anpressfläche zur Verfügung stellt. Um eine Dichtung mit einem rechteckförmigen Querschnitt einsetzen zu können, ist die Geometrie des weiblichen Kupplungsstücks gegenüber dem Camlock-Standard modifiziert. Der Abstand zwischen dem Sitz der Dichtung und Anlagefläche des männlichen Steckers bzw. die Dicke der Dichtung ist derart bemessen, dass eine ausreichende Anpresskraft erzielt wird, wenn der Stecker in das Kupplungsstück eingesetzt und mit den Verriegelungsarmen verriegelt ist. Dadurch wird eine für das Dialysekonzentrat ausreichende Dichtigkeit sichergestellt und das Wachstum von Salzkristallen im Dichtbereich vermieden, die wiederum zu Undichtigkeiten führen können. Damit wird auch der Reinigungsaufwand verringert.

Die Erfindung betrifft auch ein Dialysekonzentrat-Versorgungssystem zur Bereitstellung von Dialysekonzentrat für die Herstellung einer gebrauchsfertigen Dialysierflüssigkeit in einem Dialysezentrum oder Krankenhaus, welches eine oder mehrere Dialysekonzentrat-Einspeisevorrichtungen (nach einem der Ansprüche 10 bis 13) und ein oder mehrere Gebinde (nach Anspruch 9), wobei das Gebinde einen Faltkarton und ein Behältnis aufweist.

Darüber hinaus betrifft die Erfindung die Verwendung des Dialysekonzentrat-Versorgungssystems (nach einem der Ansprüche 10 bis 13) zum Einspeisen von Dialysekonzentrat für die Herstellung einer gebrauchsfertigen Dialysierflüssigkeit in eine oder mehrere (gebäudeseitige) Ringleitungen eines Dialysezentrums oder Krankenhauses.

Nachfolgend wird ein Ausführungsbeispiel der Erfindung unter Bezugnahme auf die Zeichnungen näher erläutert.

Es zeigen:
- Fig. 1: ein Ausführungsbeispiel des erfindungsgemäßen Behältnisses in der Seitenansicht,
- Fig. 2: das ein Basisteil, ein Einsatzteil, ein Verschlusselement und ein Deckelteil aufweisende beutelseitige Anschlussstück des Behältnisses von Fig. 1 in einer Explosionsdarstellung,
- Fig. 3: das Basisteil des beutelseitigen Anschlussstücks des Behältnisses von Fig. 1 in der Seitenansicht,
- Fig. 4: das Basisteil des beutelseitigen Anschlussstücks des Behältnisses von Fig. 1 in der Unteransicht,
- Fig. 5: das Einsatzteil des beutelseitigen Anschlussstücks des Behältnisses von Fig. 1 in der Seitenansicht,
- Fig. 6: einen Schnitt durch das Einsatzteil des beutelseitigen Anschlussstücks des Behältnisses von Fig. 1,
- Fig. 7: das beutelseitige Anschlussstück des Behältnisses von Fig. 1 in geschnittener Darstellung,
- Fig. 8: den Ausschnitt A von Fig. 7 in vergrößerter Darstellung,
- Fig. 9: ein Ausführungsbeispiel eines mit dem beutelseitigen Anschlussstück komplementären schlauchseitigen Anschlussstücks in perspektivischer Darstellung,
- Fig. 10: einen Schnitt durch das schlauchseitige Anschlussstück von Fig. 9 entlang der Linie I - I,
- Fig. 11: einen Schnitt durch das schlauchseitige Anschlussstück von Fig. 9 entlang der Linie II - II,
- Fig. 12: ein Ausführungsbeispiel des einen Faltkarton und das erfindungsgemäße Behältnis aufweisenden erfindungsgemäßen Gebindes in perspektivischer Darstellung,
- Fig. 13: eine Detailansicht des erfindungsgemäßen Gebindes in geschnittener Darstellung und
- Fig. 14: ein Ausführungsbeispiel einer Dialysekonzentrat-Versorgungsanlage mit einer Dialysekonzentrat-Einspeisevorrichtung in vereinfachter schematischer Darstellung und
- Fig. 15: eine weitere Darstellung der Dialysekonzentrat-Einspeisevorrichtung von Fig. 14.

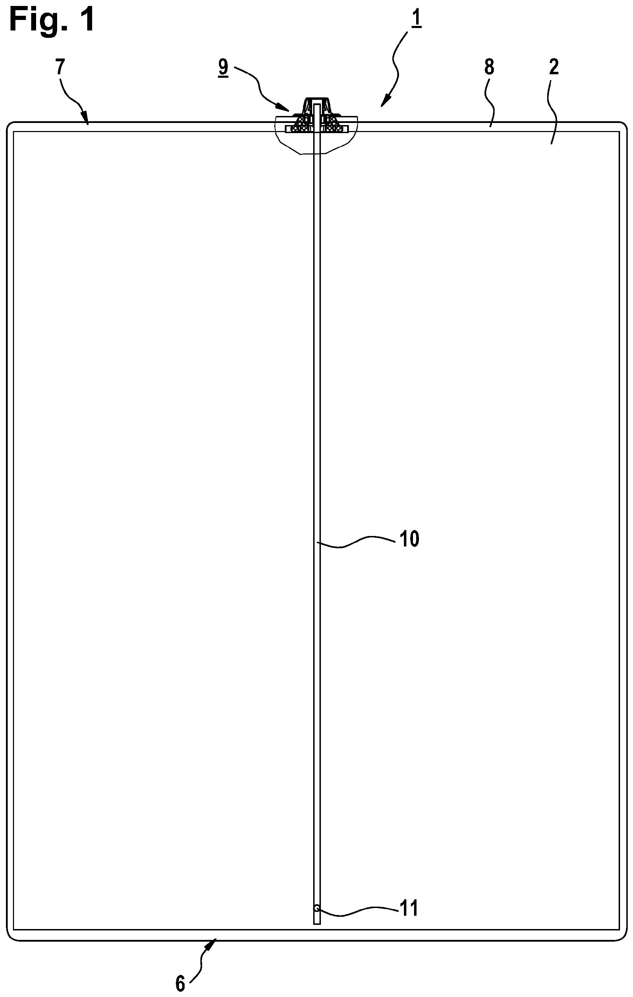

Fig. 1 zeigt in einer vereinfachten Darstellung ein Ausführungsbeispiel des erfindungsgemäßen Behältnisses 1 zur Aufnahme eines Dialysekonzentrats zur Herstellung von Dialysierflüssigkeit für die zentrale Versorgung einer Mehrzahl von in einem Dialysezentrum oder einem Krankenhaus befindlichen Blutbehandlungsvorrichtungen mit Dialysekonzentrat. Das Behältnis 1 weist einen flexiblen Folienbeutel 2 auf, welcher mit dem Dialysekonzentrat befüllt wird. Der Folienbeutel 2 ist derart dimensioniert, dass der Beutel in einen Faltkarton passend eingelegt werden kann, wenn der Folienbeutel mit Dialysekonzentrat befüllt ist. Der Folienbeutel wird aber vorzugsweise erst in den Faltkarton eingelegt und dann erst befüllt, wobei sich der Folienbeutel dabei entfalten und an die Seitenwände des Faltkartons anlegen kann.

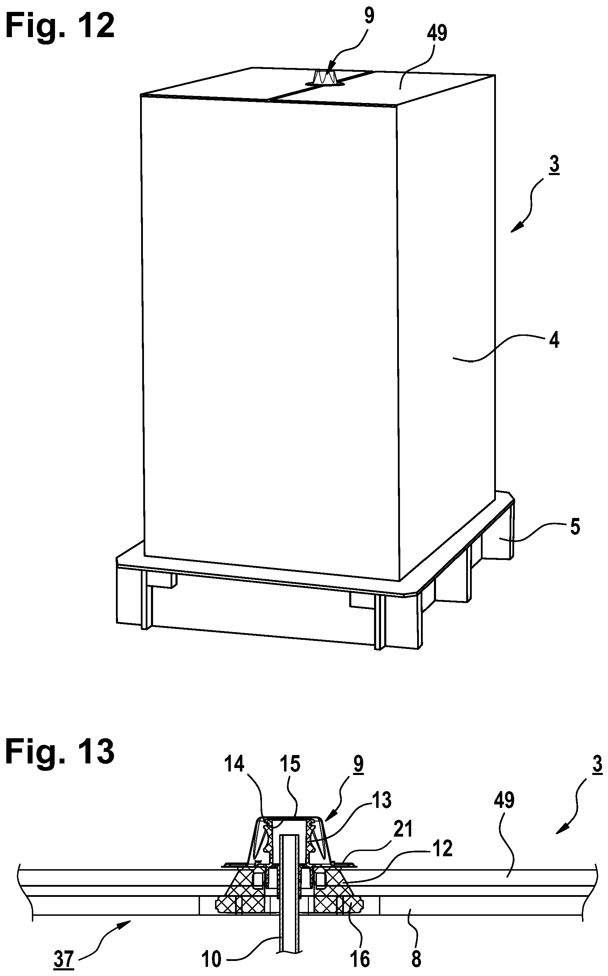

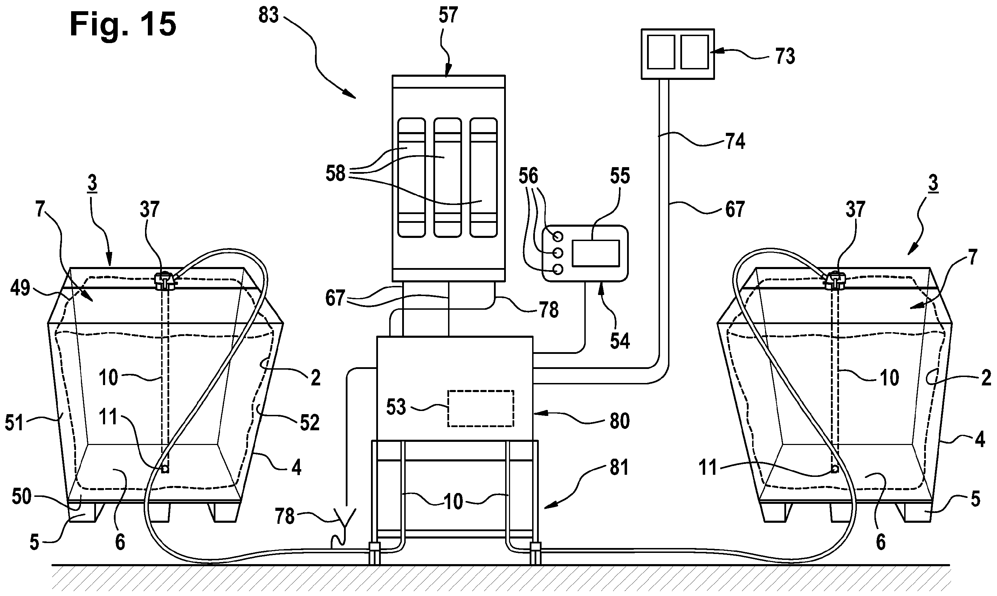

Die Figuren 12 und 15 zeigen ein Ausführungsbeispiel des erfindungsgemäßen Gebindes 3, das einen Faltkarton 4 umfasst, in den der mit Dialysekonzentrat befüllte Folienbeutel 2 passend eingelegt ist. Der Faltkarton weist einen ein oder mehrteiligen Deckelteil 49, Seitenteile 51, 52 und ein Bodenteil 50 auf. Das Gebinde 3 ist zur einmaligen Verwendung bestimmt. Aufgrund der rechteckigen Form lassen sich die Gebinde 3 auf Paletten 5 leicht transportieren. Nach dem Gebrauch können Folienbeutel 2 und Faltkarton 4 getrennt entsorgt werden. Bei dem Gebinde 3 handelt es sich um ein sogenanntes Großgebinde für eine zentrale Dialysekonzentratversorgung in einem Dialysezentrum oder Krankenhaus, bei dem der Folienbeutel 2 mehr als 50 I, vorzugsweise mehr als 200 I, beispielsweise 300 I, Dialysekonzentrat aufnehmen kann.

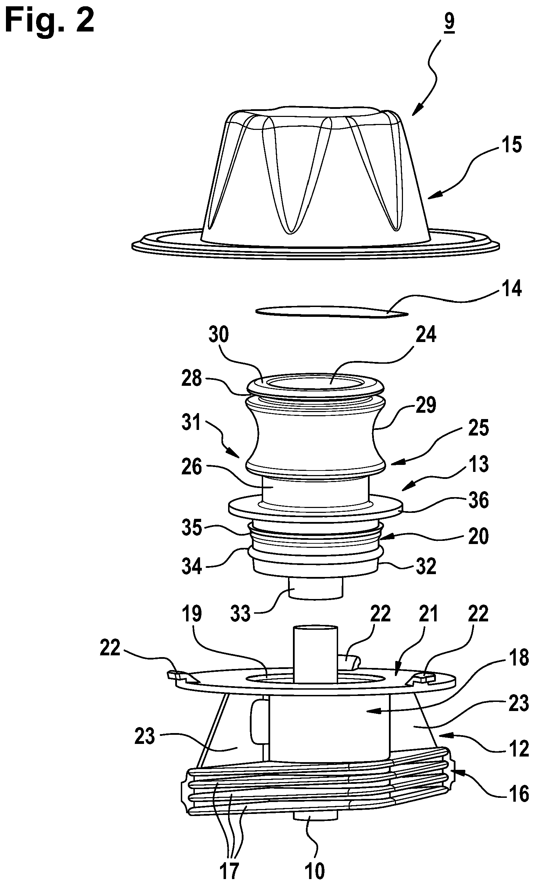

In Fig. 1 ist der Folienbeutel 1 nur vereinfacht dargestellt. Der Folienbeutel 2 weist eine in der Gebrauchslage untere Seite 6 und eine obere Seite 7 auf (Fig. 15). An der Oberseite 7 ist ein oberer Rand 8 ausgebildet, an dem zwei übereinanderliegende Folienlagen des Folienbeutels 2 miteinander dicht verschweißt sind. Der Folienbeutel 2 weist ein in den oberen Rand 8 zwischen den Folienlagen eingeschweißtes beutelseitiges Anschlussstück 9 auf, welches unter Bezugnahme auf die Figuren 2 bis 8 beschrieben wird. Das beutelseitige Anschlussstück 9 ist derart beschaffen, dass sich der Folienbeutel 2 leicht mit Dialysekonzentrat befüllen, nach der Befüllung verschließen und am Einsatzort entleeren lässt. An das beutelseitige Anschlussstück 9 ist ein sich in das Innere des Folienbeutels 2 bis zu dessen Boden erstreckender Schlauch 10 angeschlossen. An dem unteren Ende weist der Schlauch 10 eine oder mehrere seitliche Öffnungen 11 auf, um sich bei der Entnahme von Konzentrat nicht am Boden festsaugen zu können.

Fig. 2 zeigt das aus mehreren Teilen bestehende beutelseitige Anschlussstück 9 in einer perspektivischen Explosionsdarstellung vor dem Zusammenbau der Einzelteile. Das beutelseitige Anschlussstück 9 umfasst ein Basisteil 12, ein Einsatzteil 13, das mit einem Verschlusselement 14 verschlossen wird, und eine Verschlusskappe 15. Basisteil 12, Einsatzteil 13 und Verschlusskappe 15 sind Spritzgießteile, die aus thermoplastischen Kunststoffen im Spritzgießverfahren in großen Stückzahlen kostengünstig hergestellt werden können.

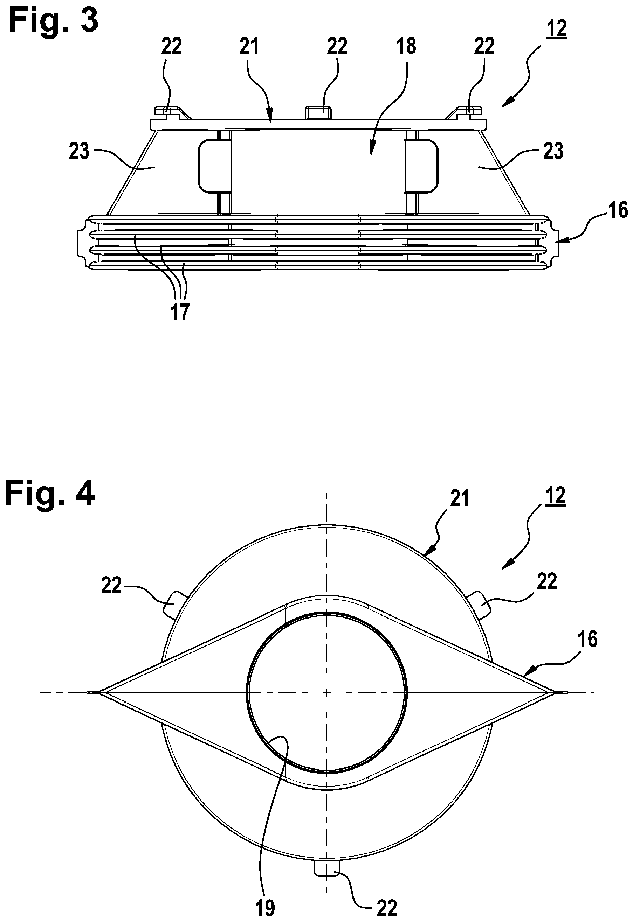

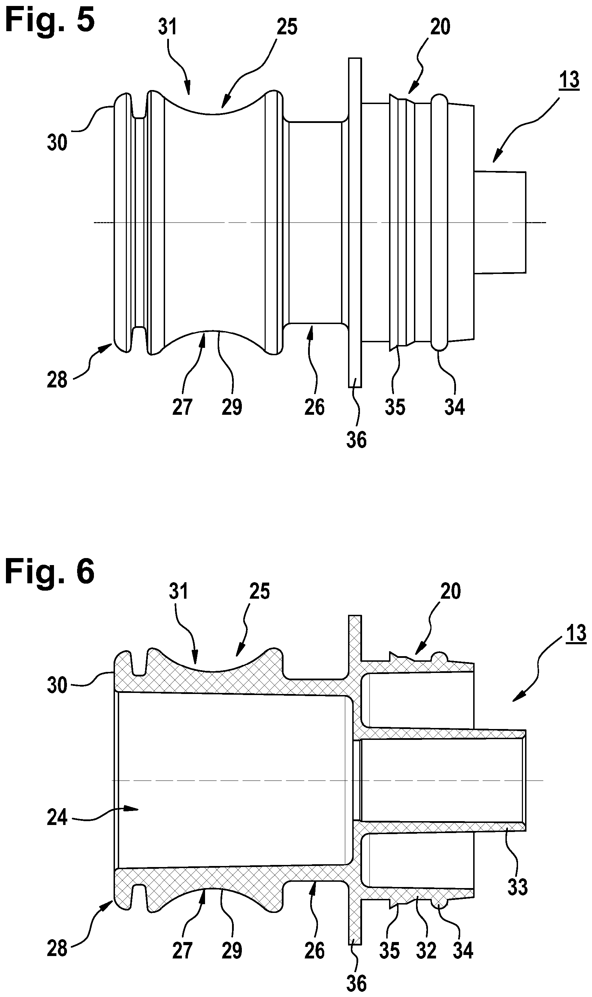

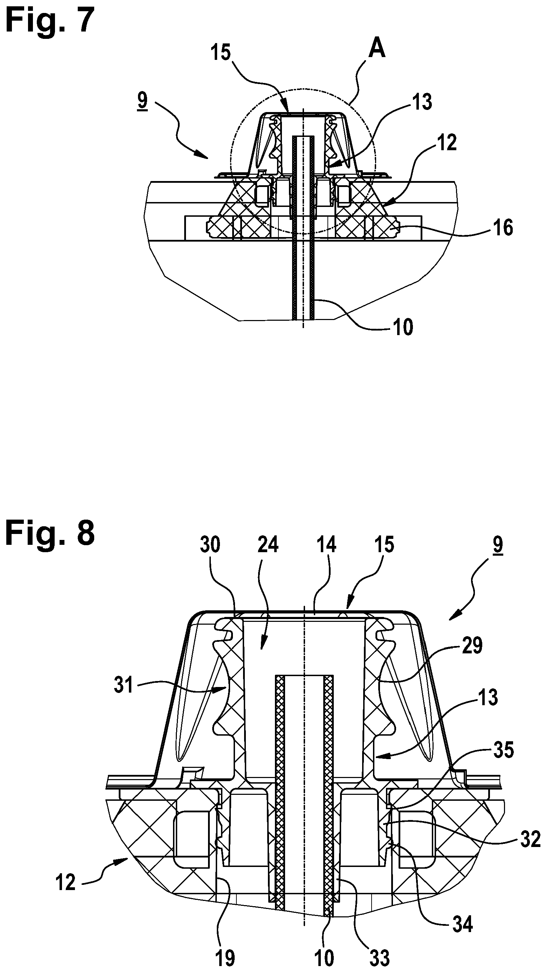

Fig. 3 zeigt das Basisteil 12 in der Seitenansicht und Fig. 4 zeigt das Basisteil 12 in der Unteransicht. Fig. 5 zeigt das Einsatzteil 13 in der Seitenansicht und Fig. 6 zeigt einen Schnitt durch das Einsatzteil 13. Fig. 7 zeigt einen Schnitt durch das Basisteil 12, Einsatzteil 13 und Verschlusskappe 15 umfassende beutelseitige Anschlussstück 9. Fig. 8 zeigt den Ausschnitt A von Fig. 7 in vergrößerter Darstellung.

Das Basisteil 12 des beutelseitigen Anschlussstücks 9 weist einen in der Art eines "Schiffchens" ausgebildeten unteren Abschnitt 16 auf, der an den Außenseiten mit umlaufenden Rippen 17 versehen ist. Der untere Abschnitt 16 des Basisteils 12 wird am oberen Rand 8 des Folienbeutels 2 derart zwischen die Innenseiten der übereinanderliegenden Folienlagen eingelegt, dass die Außenseiten des unteren Abschnitts 16 mit den Innenseiten der Folienlagen zur Anlage kommen. Dann wird das "Schiffchen" flüssigkeitsdicht mit dem Folienbeutel 2 verschweißt.

In dem unteren Abschnitt 16 und dem mittleren Abschnitt 18 des Basisteils 12 ist ein zentraler zylindrischer Durchgang 19 ausgebildet, in den ein Unterteil 20 des Einsatzteils 13 passend eingesetzt werden kann, wie nachfolgend noch erläutert wird. Der mittlere Abschnitt 18 geht in einen oberen Abschnitt über, der einen umlaufenden Flansch 21 bildet. Der umlaufende Flansch 21 weist an der Oberseite umfangsmäßig verteilt angeordnete Rastnasen 22 auf. Zwischen dem unteren Abschnitt 16 und dem oberen Flansch 21 erstrecken sich zu beiden Seiten flache Stege 23.

Das Einsatzteil 13 ist ein rotationssymmetrischer Körper mit einem zentralen Durchlass 24 (Figuren 5 und 6) und umfasst das Unterteil 20 und ein Oberteil 25.

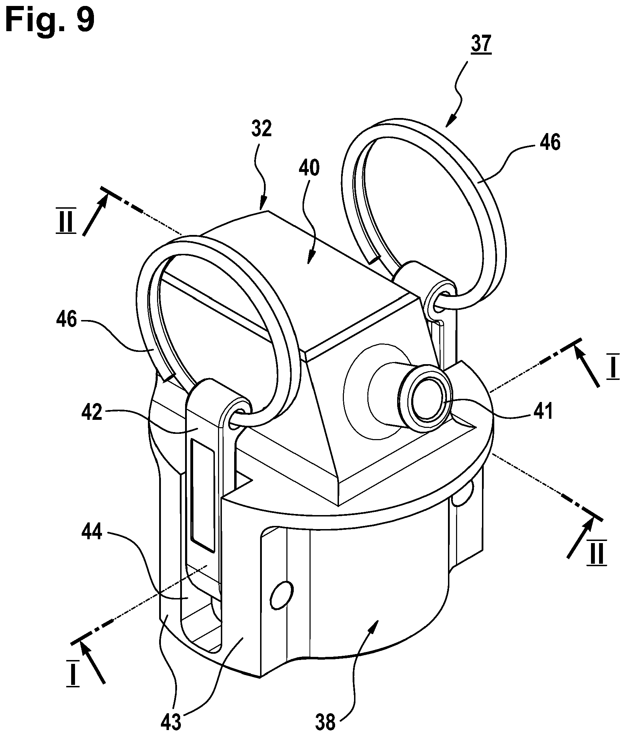

Das Oberteil 25 des Einsatzteils 13 umfasst einen unteren zylindrischen Abschnitt 26, an den sich ein verdickter mittlere Abschnitt 27 anschließt, der in einen oberen Abschnitt 28 übergeht. In dem mittleren Abschnitt 27 ist eine umlaufende Nut 29 mit einem kreisförmigen Querschnitt ausgebildet. Der obere Abschnitt 28 bildet eine äußere ringförmige obere Anlagefläche 30, die an der Oberseite weitgehend abgeflacht ist. Der zentrale Durchlass 24 hat in dem Oberteil 25 einen kreisförmigen Querschnitt. Das Oberteil 25 ist somit in der Art eines männlichen Steckers 31 einer Camlock-Kupplung ausgebildet, welcher sich passend in ein weibliches Kupplungsstück 32 der Camlock-Kupplung (Fig. 9) einstecken und in dem weiblichen Kupplungsstück verriegeln lässt, was nachfolgend noch beschrieben wird. Es sei bemerkt, dass die Abmessungen des als männlicher Stecker 31 der Camlock-Kupplung ausgebildeten Oberteils 25 des Einsatzteils 13 des beutelseitigen Anschlussstücks 9 nicht den durch Normen standardisierten Abmessungen der bekannten Camlock-Kupplungen zu entsprechen braucht, was aber zumindest für die bei der Ausbildung der Kupplung mitwirkenden Bereiche und Flächen von Vorteil sein kann.

Das Unterteil 20 des Einsatzteils 13 des beutelseitigen Anschlussstücks 9 weist ein im Wesentlichen hohlzylindrisches äußeres Einsatzstück 32 und ein im Wesentlichen hohlzylindrisches inneres Verbindungsstück 33 auf. An der Außenseite ist das Einsatzstück 32 mit Rastnasen 34 und/oder Rasthaken 35 versehen. Darüber hinaus weist das Unterteil 20 einen umlaufenden Flansch 36 auf. Das Einsatzstück 32 wird in den Durchgang 19 des Basisteils 12 eingepresst, welcher an dessen Innenseite mit einer den Rastnasen 34 und/oder Rasthaken 35 entsprechenden Profilierung versehen ist. Wenn das Einsatzteil 13 in das Basisteil 12 eingepresst ist, stützt sich der umlaufende Flansch 36 des Unterteils 20 des Einsatzteils 13 an der Oberseite des umlaufenden Flansches 21 des Basisteils 12 ab. Durch die besondere Ausbildung des Einsatzteils 12 und Basisteils 13 wird eine flüssigkeitsdichte Verbindung geschaffen.

Der Schlauch 10 wird an dem hohlzylindrischen Verbindungsstück 33 des Einsatzteils 13 angeschlossen, dessen Innendurchmesser dem Außendurchmesser des Schlauchs 10 entspricht, so dass der Schlauch in das Verbindungsstück fest eingeschoben werden kann. Dadurch wird eine ausreichende Dichtigkeit erreicht.

Das Verschlusselement 14 zum dichten Verschließen des Durchlasses 24 des Einsatzteils 13 ist eine Folie, die auf die abgeflachte Oberseite des umlaufenden Randes 30 des Oberteils 28 des Einsatzteils 13 aufgebracht wird. Die Verschlusskappe 15 ist durch die Rastnasen 22 an der Oberseite des umlaufenden Flansches 21 des Basisteils 12 fixiert und schützt die Folie vor einer Beschädigung.

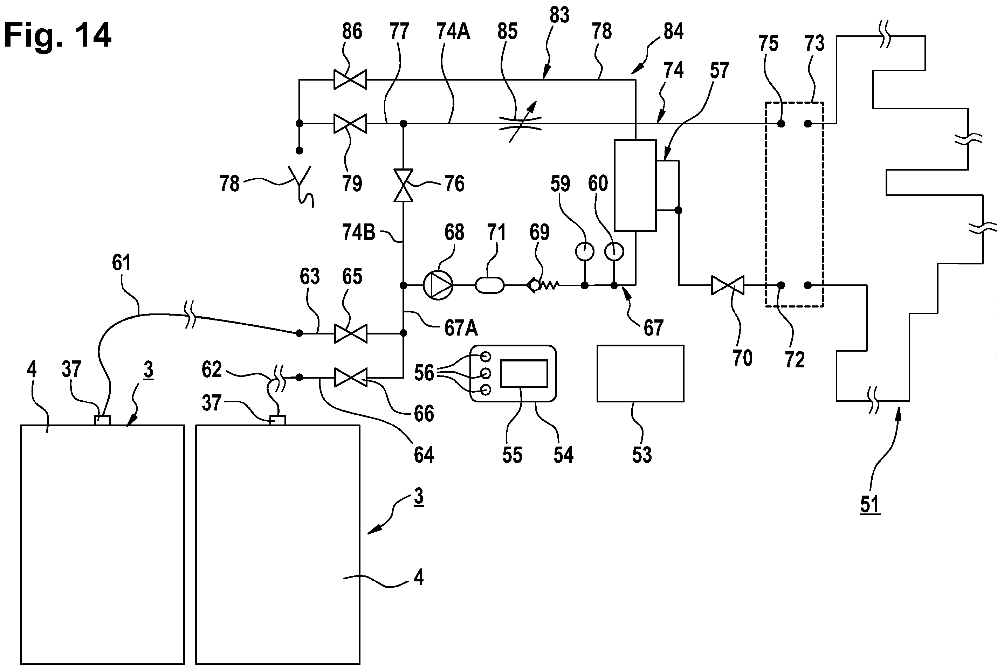

Nachfolgend wird das zu dem beutelseitigen Anschlussstück 9 komplementäre Anschlussstück 37 unter Bezugnahme auf die Figuren 9 bis 11 beschrieben. An dieses Anschlussstück 37 ist das freie Ende einer in den Figuren 9 bis 11 nicht dargestellten Schlauchleitung angeschlossen, die zu einer nicht dargestellten Dialysekonzentrat-Einspeisevorrichtung einer nicht dargestellten Dialysekonzentrat-Versorgungsanlage eines Dialysezentrums oder Krankenhauses führt. Die Dialysekonzentrat-Versorgungsanlage mit der Dialysekonzentrat-Einspeisevorrichtung wird unter Bezugnahme auf die Figuren 13 und 14 beschrieben.

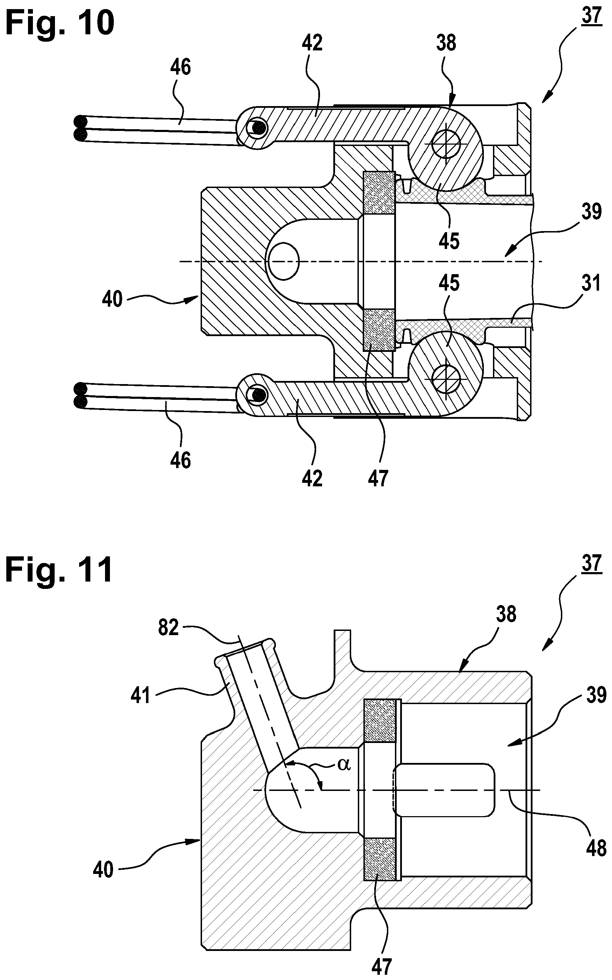

Fig. 9 zeigt das schlauchseitige Anschlussstück 37 in perspektivischer Darstellung, während Fig. 10 einen Schnitt durch das Anschlussstück 37 von Fig 9 entlang der Linie I - I und Fig. 11 einen Schnitt entlang der Linie II -II zeigt.

Das schlauchseitige Anschlussstück 37 ist als ein kappenförmiger Körper ausgebildet, der ein Basisteil 38 mit einer zentralen Ausnehmung 39 zur Aufnahme des Steckers 31 des beutelseitigen Anschlussstücks 9 und einen Deckelteil 40 mit einer Anschlusstülle 41 für eine in Fig. 9 nicht dargestellte Schlauchleitung umfasst.

Zur Verriegelung des Steckers 31 verfügt das schlauchseitige Anschlussstück 37 über zwei Verriegelungsarme 42. Das Basisteil 12 weist an zwei gegenüberliegenden Seiten seitliche Stege 43 auf, die eine Nut 44 begrenzen, wobei die Verriegelungsarme 42 an den seitlichen Stegen 43 schwenkbar befestigt sind. Die Verriegelungsarme 42 sind in einer Ebene schwenkbar befestigt, welche senkrecht auf der Ebene steht, in der die Längsachse 82 der Anschlusstülle 41 liegt. Die Verriegelungsarme 42 lassen sich zwischen einer ersten Position, in der die beiden Anschlussstücke 9 und 37 verriegelt sind, und einer zweiten Position, in der die beiden Anschlussstücke entriegelt sind, jeweils um einen Winkel von etwa 90° verschwenken.

Fig. 9 zeigt die Position, in der die beiden Verriegelungsarme 42 an dem Basisteil 12 anliegen. In dieser Position greifen die an den Verriegelungsarmen 42 vorgesehenen Nocken 45, die einen nicht kreisförmigen Querschnitt haben, fest in die umlaufende Nut 29 des als Stecker 31 ausgebildeten Oberteils 25 des Einsatzteils 13 des beutelseitigen Anschlussstücks 9, so dass die beiden Anschlussstücke 9 und 37 verriegelt sind. Wenn die Verriegelungsarme 42 nach Außen geschwenkt werden, wird die Verbindung gelöst. An den freien Enden der Verriegelungsarme 42 können Ringe 46 befestigt sein, um die Verriegelungsarme mit den Fingern besser greifen zu können.

In der Ausnehmung 39 des Basisteils 38 des schlauchseitigen Anschlussstücks 37 ist eine sich gegenüber dem Deckelteil 40 abstützende Dichtung mit einem rechteckförmigen Querschnitt 47, beispielsweise eine Ring- oder Flachdichtung, angeordnet. In der verriegelten Position wird die obere umlaufende Anlagefläche 30 des Oberteils 28 des Einsatzteils 13 gegen die Dichtung 47 gepresst, so dass eine flüssigkeitsdichte Verbindung geschaffen wird, ohne dass die Gefahr von Leckagen auch dann besteht, wenn an der Schlauchleitung gezogen wird.

Das schlauchseitige Anschlussstück 37 unterscheidet sich von dem weiblichen Kupplungsstück der bekannten in der Art einer Muffe ausgebildeten Camlock-Kupplungen, die zum Verbinden von zwei auf einer gemeinsamen Achse liegenden Schlauchleitungen bestimmt sind, durch den Deckelteil 40, der die Ausnehmung 39 des Basisteils 38 in Längsrichtung verschließt. Die Längsachse 82 der Anschlusstülle 41 an dem Deckelteil 40 des schlauchseitigen Anschlussstücks 37, an welche das freie Ende einer Schlauchleitung angeschlossen wird, ist gegenüber der Längsachse 48 der Ausnehmung 39 schräggestellt. Dabei schließt die Längsachse 82 der Anschlusstülle 41 mit der Längsachse 48 der Ausnehmung 39 einen Winkel α ein, der vorzugsweise zwischen 60-75°, insbesondere 70°, liegt, so dass die Anschlusstülle 41 in der in Fig. 11 gezeigten Darstellung im Wesentlichen nach oben weist. Wenn das Anschlussstück an einen in der Gebrauchslage befindlichen Folienbeutel angeschlossen ist, weist die Anschlusstülle 41 im Wesentlichen zur Seite. Dadurch wird die Handhabung des Anschlussstücks 37 verbessert.

Fig. 13 zeigt zur weiteren Veranschaulichung eine Detailansicht des erfindungsgemäßen Gebindes in geschnittener Darstellung. Fig. 13 zeigt, wie der umlaufende Flansch 21 des beutelseitigen Anschlussstücks 9 auf der Oberseite des Deckelteils 49 des Kartons aufliegt.

Nachfolgend wird die Dialysekonzentratversorgung in einem Dialysezentrum oder einer Klinik unter Verwendung des erfindungsgemäßen Behältnisses 1 beschrieben.

Fig. 14 zeigt in vereinfachter Darstellung ein Ausführungsbeispiel einer Dialysekonzentrat-Versorgungsanlage eines Dialysezentrums oder einer Klinik, welche grundsätzlich eine oder mehrere Dialysekonzentrat-Einspeisevorrichtungen und eine oder mehrere Ringleitungen oder Stichleitungen umfassen kann. Fig. 14 zeigt ein Ausführungsbeispiel mit nur einer Dialysekonzentrat-Einspeisevorrichtung 83 zur Einspeisung von Dialysekonzentrat in eine einzige gebäudeseitige Ringleitung 51. Die Dialysekonzentrat-Einspeisevorrichtung 83 befindet sich in einem Raum, in dem auch die mit Dialysekonzentrat befüllten Behältnisse 1 aufgestellt werden. An die Dialysekonzentrat-Einspeisevorrichtung 83 können mehrere Großgebinde 3 angeschlossen werden. Dadurch kann eine unterbrechungsfreie Versorgung mit Dialysekonzentrat ermöglicht werden, weil ein leeres Gebinde durch ein volles Gebinde an einem Anschluss ersetzt werden kann, während über einen anderen Anschluss Dialysekonzentrat angesaugt werden kann. Fig. 15 zeigt zur besseren Veranschaulichung nochmals die einzelnen Komponenten der Dialysekonzentrat-Einspeisevorrichtung 83 in ihrer räumlichen Umgebung, wobei die einander entsprechenden Teile mit denselben Bezugszeichen versehen sind. Einzelne Aggregate oder Komponenten der Dialysekonzentrat-Einspeisevorrichtung 83 können in einem Gehäuse 80 angeordnet sein, welches auf einem verfahrbaren Wagen 81 stehen kann.

Die Dialysekonzentrat-Einspeisevorrichtung 83 umfasst ein Leitungssystem 84, eine Anordnung von Elementen zur Steuerung des Flüssigkeitsflusses im Leitungssystem 84, beispielsweise Ventile oder Drosseln, eine oder mehrere Pumpen, eine Steuereinrichtung 53 zur Ansteuerung der einzelnen Komponenten sowie eine Benutzerschnittstelle 54 (Benutzerschnittstelle). Die Ventile sind vorzugsweise elektromagnetisch betätigbare Ventile, die von der Steuereinrichtung 53 angesteuert werden und/oder manuell betätigt werden können. Die Benutzerschnittstelle 54 kann eine Einheit bilden, welche an der Wand eines Raums befestigt werden kann, um leicht zugänglich zu sein. Die Benutzerschnittstelle 54 kann einen Bildschirm 55 sowie Bedienelemente 56, beispielsweise Schalter oder Taster, umfassen. Der Bildschirm 55 kann als Touchscreen ausgebildet sein. Darüber hinaus können noch weitere Komponenten vorgesehen sein, beispielsweise eine Filtereinrichtung 57 (Ultrafilter) mit mehreren Filterkartuschen 58 und/oder ein oder mehrere Druckmesser 59, 60 für eine Überwachung des Drucks in dem Leitungssystem 84. Die Filtereinrichtung 57 mit den Kartuschen 58 kann auch eine separate Einheit bilden, die an der Wand des Raums aufgehängt werden kann.

Bei dem vorliegenden Ausführungsbeispiel verfügt das Leitungssystem 84 der Dialysekonzentrat-Einspeisevorrichtung 83 über zwei Dialysekonzentrat-Anschlussleitungen 61, 62, bei denen es sich um flexible Schlauchleitungen handelt, welche an den freien Enden mit dem erfindungsgemäßen schlauchseitigen Anschlussstück 37 versehen sind, welches an das beutelseitige Anschlussstück 9 des Großgebindes 3 angeschlossen wird. Die Dialysekonzentrat-Anschlussleitungen 61, 62 führen zu zwei Einspeiseleitungen 63, 64, in denen jeweils ein Absperrventil 65, 66 angeordnet ist, um die Leitungen 63, 64 absperren zu können. Zum Einspeisen von Dialysekonzentrat aus dem Großgebinde 3 öffnet die Steuereinrichtung 53 das eine Ventil 65 bzw. 66 und schließt das andere Ventil 66 bzw. 65. Die Ventile 65, 66 können aber auch von Hand betätigbare Ventile sein. Die beiden Einspeiseleitungen 63, 64 gehen in eine gemeinsame Dialysekonzentratzuführleitung 67 über, in welcher eine Pumpe 68 zum Fördern des Konzentrats angeordnet ist. In der Dialysekonzentratzuführleitung 67 ist stromab der Pumpe 68 ein Rückschlagventil 69 und eine Filtereinrichtung 57 (Ultrafilter) sowie ein Ventil 70 zum Absperren der Leitung 67 angeordnet. Wenn die Pumpe 68 eine Membranpumpe ist, kann stromab der Pumpe 68 noch ein Pulsationsdämpfer 71 vorgesehen sein.

Die Dialysekonzentratzuführleitung 67 wird an den Zulaufanschluss 72 einer in dem Raum vorgesehenen Anschlussstelle 73 angeschlossen, um die Leitung 67 an die gebäudeseitige Ringleitung 51 anzuschließen. Darüber hinaus umfasst das Leitungssystem 84 eine Rücklauflaufleitung 74, die den Ablaufanschluss 75 der Anschlussstelle 73 mit einem Leitungsabschnitt 67A der Zuführleitung 67 stromauf der Pumpe 68 verbindet. Die Rücklauflaufleitung 74 umfasst einen ersten Abschnitt 74A, in dem eine einstellbare Drossel 85 angeordnet ist, und einen zweiten Abschnitt 74B, in dem ein Ventil 76 angeordnet ist. Von dem Leitungsabschnitt der Rücklauflaufleitung 74 zwischen der Drossel 85 und dem Ventil 76 zweigt von der Rücklauflaufleitung 74 eine Abflussleitung 77 ab, die zu einem Ablauf 78 führt, in welcher ein Ventil 79 angeordnet ist. Wenn das Ventil 70 in der Zuführleitung 67 und das Ventil 76 in der Rücklauflaufleitung 74 geöffnet und das Ventil 79 in der Abflussleitung 77 geschlossen sind, wird Dialysekonzentrat durch die Ringleitung 51 gepumpt. Der Druck in der Ringleitung 51 kann mit der Drossel 85 oder softwareseitig mittels des Druckmessers 59 eingestellt werden. Zum Spülen oder Desinfizieren wird das Ventil 79 in der Abflussleitung 77 geöffnet. Zum Spülen der Filtereinrichtung 57 kann eine von der Filtereinrichtung 57 zu dem Ablauf 78 führende Spülleitung 78 vorgesehen sein, in welcher ein Ventil 86 angeordnet ist.

Die erfindungsgemäßen Anschlusstücke 9, 37 erlauben eine einfache und sichere Handhabung der Großgebinde. Die Anschlusstücke 9, 37 können erst dann verriegelt werden, wenn sie zueinander korrekt ausgerichtet sind. Dadurch ist ein Verkanten der Teile, was zu Undichtigkeiten führen könnte, ausgeschlossen. Beim Umlegen der Arretierungsarme 42 des schlauchseitigen Anschlussstücks 37 werden keine vertikalen Druckkräfte auf das Gebinde 3 ausgeübt, wodurch das Gebinde eingedrückt werden könnte. Nach dem Umlegen der Arretierungsarme wird der Stecker 31 des beutelseitigen Anschlussstücks 9 fest gegen die Dichtung 47 des beutelseitigen Anschlussstücks 37 gepresst. Die dichte Verbindung verhindert das unbemerkte Ansaugen von Luft, die zu Problemen bei der Verteilung des Konzentrats und zu Schäden an der Pumpe 68 (Trockenlaufen) führen könnte. Die seitlich anliegenden Arretierungsarme 42 lassen sofort erkennen, dass eine dichte Verbindung hergestellt ist.

Da sich der Anschluss an der Oberseite des Gebindes 3 befindet, besteht bei Schäden an der Kupplung oder falschem Handling nicht die Gefahr des Auslaufens von Konzentrat. Auch kann beim Lösen der Verbindung keine Flüssigkeit auslaufen, wenn sich noch eine Restmenge in dem Folienbeutel 2 befinden sollte. Darüber hinaus bietet der Anschluss an der Beuteloberseite ergonomische Vorteile, da er leichter zu erreichen ist.

Die Schlauchleitung 61, 62 der Dialysekonzentrat-Einspeisevorrichtung 83 kann eine dünne, flexible Schlauchleitung sein, die beim Anschluss des Gebindes 3 nicht ohne weiteres abknicken kann. Da die Anschlusstülle 41 des schlauchseitigen Anschlussstücks 37 leicht nach oben zeigt, lässt sich der Schlauch so führen, dass er beim Anschluss des Gebindes nicht stört.

Die Konzeption des erfindungsgemäßen Behältnisses ermöglicht ein vereinfachtes Herstellungsverfahren, das durch die folgenden Verfahrensschritte gekennzeichnet ist:
a) Bereitstellen eines Folienbeutels 2 aus einer flexiblen Folie,
b) Bereitstellen eines beutelseitigen Anschlussstücks 9 zum Anschluss eines schlauchleitungsseitigen Anschlussstücks 37 einer Schlauchleitung 61, 62 einer Dialysekonzentrat-Einspeisevorrichtung 83, wobei das beutelseitige Anschlussstück ein Basisteil 12 aufweist, welches einen Durchgang 19 aufweist,
c) Bereitstellen eines Einsatzteils 13, welches einen Durchlass 24 für das Dialysekonzentrat aufweist, wobei das Einsatzteil 13 ein als männlicher Stecker 31 einer Camlock-Kupplung ausgebildetes Oberteil 25 mit einer umlaufenden Nut 29 und ein Unterteil 20 aufweist, welches ein im Wesentlichen hohlzylindrisches äußeres Einsatzstück 32, und ein im Wesentlichen hohlzylindrisches inneres Verbindungsstück 33 umfasst,
d) flüssigkeitsdichtes Verbinden des Basisteils 12 des beutelseitigen Anschlussstücks 9 mit den Folienlagen des Folienbeutels 2 an der in der Gebrauchslage oberen Seite 7 des Folienbeutels 2,
e) flüssigkeitsdichtes Verschließen des Oberteils 25 des Einsatzteils 13 mit einem abnehmbaren oder abtrennbaren Verschlusselement 14, insbesondere eine abziehbare Folie,
f) Anschließen eines Schlauchs 10 an das im Wesentlichen hohlzylindrische innere Verbindungsstück 33 des Unterteils 20 des Einsatzteils 13,
g) Befüllen des Folienbeutels 2 mit Dialysekonzentrat,
h) Einführen des Schlauchs 10 in den Folienbeutel 2 und Einsetzen des im Wesentlichen hohlzylindrischen äußeren Einsatzstücks 32 des Unterteils 20 des Einsatzteils 13 in den Durchgang 19 des Basisteils 12 des beutelseitigen Anschlussstücks 9.

Die einzelnen Verfahrensschritte a) bis h) können gleichzeitig oder in unterschiedlicher Reihenfolge erfolgen, sofern nicht ein Verfahrensschritt die Durchführung eines anderen Verfahrensschritts zwingend voraussetzt. Die einzelnen Verfahrensschritte a) bis h) können auch an unterschiedlichen Orten ausgeführt werden. Beispielsweise können die Verfahrensschritte a) bis f) zur Bereitstellung der einzelnen Teile an einem ersten Ort und die Verfahrensschritte g) und h) zum Befüllen des Behältnisses an einem zweiten Ort erfolgen. Der Verfahrensschritt e) des flüssigkeitsdichten Verschließens des Oberteils 25 des Einsatzteils 13 mit einem abnehmbaren oder abtrennbaren Verschlusselement 14, insbesondere eine abziehbare Folie, ist zwar für die Produktion von Vorteil, aber nicht zwingend erforderlich.

Wenn der Beutel in einen Faltkarton mit einem Bodenteil, Seitenteilen und einem Deckelteil bereitgestellt werden soll, wird das Behältnis derart in den aufgestellten Faltkarton eingesetzt, dass der Folienbeutel 2 auf dem Bodenteil 50 des Faltkartons 4 aufsteht und sich das Oberteil 25 des Einsatzteils 13 durch den Deckelteil 49 nach außen erstreckt. Das erfindungsgemäße Behältnis erlaubt ein Befüllen des in den Faltkarton 4 eingesetzten Behältnisses, was in der Praxis von Vorteil ist, wobei die Verfahrensschritte g) und h) erst dann durchgeführt werden, wenn der Beutel in den Faltkarton eingesetzt worden ist.

## Patentansprüche

1. Behältnis zum Anschluss an eine Schlauchleitung einer Dialysekonzentrat-Einspeisevorrichtung zur Bereitstellung von Dialysekonzentrat für die Herstellung einer gebrauchsfertigen Dialysierflüssigkeit in einem Dialysezentrum oder Krankenhaus, wobei das Behältnis (1) einen Folienbeutel (2) aus einer flexiblen Folie aufweist, welcher eine in der Gebrauchslage untere Seite (6) und eine obere Seite (7) aufweist und ein Innenvolumen zur Aufnahme des flüssigen Dialysekonzentrats umschließt, **dadurch gekennzeichnet, dass**
an der Oberseite (7) des Folienbeutels ein beutelseitiges Anschlussstück (9) vorgesehen ist, welches zum Anschluss eines schlauchleitungsseitigen Anschlussstücks (37) der Schlauchleitung (61, 62) ausgebildet ist, wobei das beutelseitige Anschlussstück (9) ein Basisteil (12) aufweist, welches an der Oberseite (7) des Folienbeutels mit den Folienlagen flüssigkeitsdicht verbunden ist, und das Basisteil (12) einen Durchgang (19) aufweist,
das beutelseitige Anschlussstück (9) einen in den Durchgang (19) des Basisteils (12) eingesetztes Einsatzteil (13) aufweist, welches einen Durchlass (24) für das Dialysekonzentrat aufweist, und
das Einsatzteil (13) ein in der Art eines männlichen Steckers einer Camlock-Kupplung ausgebildetes Oberteil (25) mit einer umlaufenden Nut (29) und ein Unterteil (20) umfasst, welches ein im Wesentlichen hohlzylindrisches äußeres Einsatzstück (32), das in den Durchgang (19) des Basisteils (12) eingesetzt ist, und ein im Wesentlichen hohlzylindrisches inneres Verbindungsstück (33) aufweist, an welches ein sich in das Innenvolumen des Folienbeutels (2) erstreckender Schlauch (10) angeschlossen ist, und
wobei das Unterteil (20) des Einsatzteils (13) und das Oberteil (25) des Einsatzteils einstückig sind.

2. Behältnis nach Anspruch 1, **dadurch gekennzeichnet, dass** das im Wesentlichen hohlzylindrische äußere Einsatzstück (32) des Unterteils (25) des Einsatzteils (13) einrastend und/oder klemmend in den Durchgang (19) des Basisteils (12) des beutelseitigen Anschlussstücks (9) eingepresst ist.

3. Behältnis nach Anspruch 2, **dadurch gekennzeichnet, dass** das obere Endstück des Schlauchs (10) in das im Wesentlichen hohlzylindrische innere Verbindungsstück (33) des Unterteils (25) des Einsatzteils (13) eingeschoben ist.

4. Behältnis nach einem der Ansprüche 1 bis 3, **dadurch gekennzeichnet, dass** das Basisteil (12) einen oberen umlaufenden Flansch (21) aufweist.

5. Behältnis nach einem der Ansprüche 1 bis 4, **dadurch gekennzeichnet, dass** das Einsatzteil (13) einen umlaufenden Rand (36) aufweist, der sich auf der Oberseite des umlaufenden Flansches (21) des Basisteils (12) abstützt.

6. Behältnis nach einem der Ansprüche 1 bis 5, **dadurch gekennzeichnet, dass** auf das Oberteil (25) des Einsatzteils (13) eine Verschlusskappe (15) aufgesetzt ist.

7. Behältnis nach einem der Ansprüche 1 bis 6, **dadurch gekennzeichnet, dass** das Behältnis (1) mit einem flüssigen Dialysekonzentrat, insbesondere mit einem Säurekonzentrat, zur Herstellung einer gebrauchsfertigen Dialysierflüssigkeit befüllt ist.

8. Behältnis nach einem der Ansprüche 1 bis 7, **dadurch gekennzeichnet, dass** das Basisteil (12) und das Einsatzteil (13) des beutelseitigen Anschlussstücks (9) im Spritzgießverfahren hergestellte Spritzgießteile sind.

9. Gebinde aufweisend einen Faltkarton (4) mit einem Bodenteil (50), Seitenteilen (51, 52) und einem Deckelteil (49) und einem Behältnis (1) nach einem der Ansprüche 1 bis 8, **dadurch gekennzeichnet, dass** das Behältnis (1) in den Faltkarton (4) derart eingesetzt ist, dass der Folienbeutel (1) auf dem Bodenteil (50) aufsteht, wobei sich das Oberteil (25) des Einsatzteils (13) durch den Deckelteil (49) nach außen erstreckt.

10. Dialysekonzentrat-Einspeisevorrichtung zur Bereitstellung von Dialysekonzentrat für die Herstellung einer gebrauchsfertigen Dialysierflüssigkeit in einem Dialysezentrum oder Krankenhaus, wobei die Dialysekonzentrat-Einspeisevorrichtung (83) mindestens eine Schlauchleitung (61, 62) zum Ansaugen von Dialysekonzentrat aufweist, an deren freiem Ende ein Anschlussstück (37) zur Herstellung einer Flüssigkeitsverbindung mit einem Anschlussstück (9) eines mit Dialysekonzentrat befüllten Behältnisses (1) vorgesehen ist,
**dadurch gekennzeichnet, dass**
das Anschlussstück (37) der Schlauchleitung (61, 62) in der Art eines weiblichen Kupplungsstücks einer Camlock-Kupplung ausgebildet ist, welches zur Aufnahme eines in der Art eines männlichen Steckers (31) der Camlock-Kupplung ausgebildeten Steckers ausgebildet ist, und welches zwei Verriegelungsarme (42) zur Verriegelung des männlichen Steckers aufweist, wobei das weibliche Kupplungsstück der Camlock-Kupplung als ein kappenförmiger Körper ausgebildet ist, welcher ein Basisteil (38), in dem eine Ausnehmung (39) vorgesehen ist, und einen Deckelteil (40) aufweist, wobei der Deckelteil (40) mit einer Anschlusstülle (41) versehen ist, an welche das freie Ende der Schlauchleitung (61, 62) angeschlossen ist.

11. Dialysekonzentrat-Einspeisevorrichtung nach Anspruch 10, **dadurch gekennzeichnet, dass** die Längsachse (82) der Anschlusstülle (41) gegenüber der Längsachse (48) der Ausnehmung (39) schräggestellt ist.

12. Dialysekonzentrat-Einspeisevorrichtung nach Anspruch 10 oder 11, **dadurch gekennzeichnet, dass**
die Verriegelungsarme (42) an gegenüberliegenden Seiten des Basisteils (38) in einer Ebene schwenkbar befestigt sind, welche senkrecht auf der Ebene steht, in der die Längsachse (82) der Anschlusstülle (41) liegt, und/oder das Basisteil (38) an gegenüberliegenden Seiten seitliche Stege (43) aufweist, die eine Nut (44) begrenzen, wobei die Verriegelungsarme (42) an den seitlichen Stegen (43) schwenkbar befestigt sind.

13. Dialysekonzentrat-Einspeisevorrichtung nach einem der Ansprüche 10 bis 12, **dadurch gekennzeichnet, dass** in der Ausnehmung (39) des Basisteils (38) eine sich gegenüber dem Deckelteil (40) abstützende Dichtung mit einem im Wesentlichen rechteckigen Querschnitt (47) angeordnet ist.

14. Verfahren zur Herstellung eines Behältnisses zum Anschluss an eine Schlauchleitung einer Dialysekonzentrat-Einspeisevorrichtung zur Bereitstellung von Dialysekonzentrat für die Herstellung einer gebrauchsfertigen Dialysierflüssigkeit in einem Dialysezentrum oder Krankenhaus, mit folgenden Verfahrensschritten:
Bereitstellen eines Folienbeutels (2) aus einer flexiblen Folie,
Bereitstellen eines mit dem Folienbeutel zu verbindenden, beutelseitigen Anschlussstücks (9) zum Anschluss eines schlauchleitungsseitigen Anschlussstücks (37) einer Schlauchleitung (61, 62) einer Dialysekonzentrat-Einspeisevorrichtung (83), wobei das beutelseitige Anschlussstück (9) ein Basisteil (12) aufweist, welches einen Durchgang (19) aufweist,
Bereitstellen eines Einsatzteils (13), welches einen Durchlass (24) für das Dialysekonzentrat aufweist, wobei das Einsatzteil (13) ein als männlicher Stecker einer Camlock-Kupplung ausgebildetes Oberteil (25) mit einer umlaufenden Nut (27) und ein Unterteil (20) aufweist, welches ein im Wesentlichen hohlzylindrisches äußeres Einsatzstück (32), und ein im Wesentlichen hohlzylindrisches inneres Verbindungsstück (33) umfasst,
flüssigkeitsdichtes Verbinden des Basisteils (12) des beutelseitigen Anschlussstücks (9) mit den Folienlagen des Folienbeutels (2) an der in der Gebrauchslage oberen Seite (7) des Folienbeutels (2),
flüssigkeitsdichtes Verschließen des Oberteils (25) des Einsatzteils (13) mit einem abnehmbaren oder abtrennbaren Verschlusselement (14), insbesondere eine abziehbare Folie,
Anschließen eines Schlauchs (10) an das im Wesentlichen hohlzylindrische innere Verbindungsstück (33) des Unterteils (20) des Einsatzteils (13),
Befüllen des Folienbeutels (2) mit Dialysekonzentrat,
Einführen des Schlauchs (10) in den Folienbeutel (2) und Einsetzen des im Wesentlichen hohlzylindrischen äußeren Einsatzstücks (32) des Unterteils (20) des Einsatzteils (13) in den Durchgang (19) des Basisteils (12) des beutelseitigen Anschlussstücks (9).

15. Verfahren nach Anspruch 14, **dadurch gekennzeichnet, dass** der Folienbeutel (2) vor dem Befüllen in einen Faltkarton (4) mit einem Bodenteil (50), Seitenteilen (51, 52) und einem Deckelteil (7) derart eingesetzt wird, dass der Folienbeutel (2) auf dem Bodenteil (50) aufsteht und sich das Oberteil (25) des Einsatzteils (13) des beutelseitigen Anschlussstücks (9) durch den Deckelteil (7) nach außen erstreckt.

## Claims

1. Container for connection to a hose conduit of a dialysis concentrate supply device for providing dialysis concentrate to produce a ready-to-use dialysis fluid in a dialysis centre or hospital, wherein the container (1) has a film bag (2) made of a flexible film, which has a lower side (6) in the position of use and an upper side (7) and encloses an internal volume for receiving the liquid dialysis concentrate,
**characterised in that**
a connection piece (9) on the bag side is provided on the upper side (7) of the film bag, which is designed for connecting a connection piece (37) of the hose conduit (61, 62) on the hose conduit side, wherein the connection piece (9) on the bag side has a base part (12), which is connected to the film layers in a liquid-tight manner on the upper side (7) of the film bag, and the base part (12) has a passage (19),
the connection piece (9) on the bag side has an insert part (13), which is inserted into the passage (19) of the base part (12) and has a passage (24) for the dialysis concentrate, and
the insert part (13) comprises an upper part (25) designed in the manner of a male connector for a camlock coupling with a circumferential groove (29) and a lower part (20), which has a substantially hollow cylindrical outer insert piece (32), which is inserted into the passage (19) of the base part (12), and a substantially hollow cylindrical inner connection piece (33), to which a hose (10) extending into the inner volume of the film bag (2) is connected, and
wherein the lower part (20) of the insert part (13) and the upper part (25) of the insert part are in one piece.

2. Container according to claim 1, **characterised in that** the substantially hollow-cylindrical outer insert piece (32) of the lower part (25) of the insert part (13) is pressed into the passage (19) of the base part (12) of the connection piece (9) on the bag side in a latching and/or clamping manner.

3. Container according to claim 2, **characterised in that** the upper end piece of the tube (10) is inserted into the substantially hollow-cylindrical inner connection piece (33) of the lower part (25) of the insert part (13).

4. Container according to any of claims 1 to 3, **characterised in that** the base part (12) has an upper circumferential flange (21).

5. Container according to any of claims 1 to 4, **characterised in that** the insert part (13) has a circumferential edge (36), which is supported on the upper side of the circumferential flange (21) of the base part (12).

6. Container according to any of claims 1 to 5, **characterised in that** a closure cap (15) is placed on the upper part (25) of the insert part (13).

7. Container according to any of claims 1 to 6, **characterised in that** the container (1) is filled with a liquid dialysis concentrate, particularly with an acid concentrate, to produce a ready-to-use dialysis concentrate.

8. Container according to any of claims 1 to 7, **characterised in that** the base part (12) and the insert part (13) of the bag-side connection piece (9) are injection moulding parts produced by the injection moulding method.

9. Pack having a folding carton (4) with a base part (50), side parts (51, 52) and a lid part (49) and a container (1) according to one of claims 1 to 8, **characterised in that** the container (1) is inserted into the folding carton (4) in such a manner that the film bag (1) stands up on the base part (50), wherein the upper part (25) of the insert part (13) extends outwards through the lid part (49).

10. Dialysis concentrate supply device for providing dialysis concentrate to produce a ready-to-use dialysis fluid in a dialysis centre or hospital, wherein the dialysis concentrate supply device (83) has at least one hose conduit (61, 62) for drawing in dialysis concentrate, at the free end of which a connection piece (37) is provided to produce a fluid connection with a connection piece (9) of a container (1) filled with dialysis concentrate,
**characterised in that**
the connection piece (37) of the hose conduit (61, 62) is designed in the manner of a female coupling piece of a camlock coupling, which is designed for receiving a connector designed in the manner of a male connector (31) of the camlock coupling, and which has two locking arms (42) for locking the male connector wherein the female coupling piece of the camlock coupling is designed as a cap-shaped body, which has a base part (38), in which a cut-out (39) is provided, and a lid part (40), wherein the lid part (40) is provided with a connecting grommet (41), to which the free end of the hose conduit (61, 62) is connected.

11. Dialysis concentrate supply device according to claim 10, **characterised in that** the longitudinal axis (82) of the connecting grommet (41) is inclined relative to the longitudinal axis (48) of the cut-out (39).

12. Dialysis concentrate supply device according to claim 10 or 11, **characterised in that**
the locking arms (42) are fastened pivotably to opposite sides of the base part (38) in a plane that is perpendicular to the plane in which the longitudinal axis (82) of the connecting grommet (41) lies, and/or
the base part (38) has lateral ridges (43) on opposite sides, which delimit a groove (44), wherein the locking arms (42) are pivotably fastened to the lateral ridges (43).

13. Dialysis concentrate supply device according to any of claims 10 to 12, **characterised in that** a seal with a substantially rectangular cross-section (47) is arranged in the cut-out (39) of the base part (38) and is supported relative to the lid part (40).

14. Method for manufacturing a container for connection to a hose conduit of a dialysis concentrate supply device for providing dialysis concentrate to produce a ready-to-use dialysis fluid in a dialysis centre or hospital, comprising the following method steps:
providing a film bag (2) made of a flexible film,
providing a connection piece (9) on the bag side to be connected to the film bag for connecting a connection piece (37) on the hose conduit side of a hose conduit (61, 62) of a dialysis concentrate supply device (83), wherein the connection piece (9) on the bag side has a base part (12) which has a passage (19),
providing an insert piece (13) which has a passage (24) for the dialysis concentrate, wherein the insert part (13) comprises an upper part (25) designed as a male connector of a camlock coupling with a circumferential groove (27) and a lower part (20), which comprises a substantially hollow-cylindrical outer insert piece (32) and a substantially hollow-cylindrical inner connection piece (33),
liquid-tight connection of the base part (12) of the bag-side connection piece (9) to the film layers of the film bag (2) on the upper side (7) of the film bag (2) in the position of use,
liquid-tight closing of the upper part (25) of the insert part (13) with a removable or detachable closure element (14), in particular a removable film
connecting a hose (10) to the substantially hollow cylindrical inner connection piece (33) of the lower part (20) of the insert part (13),
filling the film bag (2) with dialysis concentrate,
inserting the tube (10) into the film bag (2) and inserting the substantially hollow cylindrical outer insert piece (32) of the lower part (20) of the insert part (13) into the passage (19) of the base part (12) of the connection piece (9) on the bag side.

15. Method according to claim 14, **characterised in that**, before filling, the film bag (2) is inserted into a folding carton (4) with a base part (50), side parts (51, 52) and a lid part (7) in such a manner that the film bag (2) stands up on the base part (50) and the upper part (25) of the insert part (13) of the bag-side connection piece (9) extends outwards through the lid part (7).

## Revendications

1. Récipient destiné à être raccordé à une conduite en tuyau flexible d'un dispositif d'alimentation en concentré de dialyse afin de fournir du concentré de dialyse pour la préparation d'un liquide de dialyse prêt à l'emploi dans un centre de dialyse ou un hôpital, le récipient (1) comportant une poche en film (2) constituée d'un film souple, laquelle présente une face inférieure (6) et une face supérieure (7) en position d'utilisation et qui renferme un volume intérieur destiné à recevoir le concentré de dialyse liquide,
**caractérisé en ce que**
un élément de raccordement côté poche (9) est prévu sur la face supérieure (7) de la poche en film, lequel est conçu pour le raccordement d'un élément de raccordement côté tuyau (37) de la conduite en tuyau flexible (61, 62), l'élément de raccordement côté poche (9) comportant une partie de base (12) qui est reliée de manière étanche aux liquides aux couches de film sur la face supérieure (7) de la poche en film, et la partie de base (12) comportant un passage (19),
l'élément de raccordement côté poche (9) comporte un insert (13) inséré dans le passage (19) de la partie de base (12) et muni d'un orifice (24) pour le concentré de dialyse, et
l'insert (13) comprend une partie supérieure (25) conçue à la manière d'une fiche mâle d'un raccord Camlock, dotée d'une rainure périphérique (29), et une partie inférieure (20) qui comporte un élément d'insertion extérieur sensiblement cylindrique creux (32), inséré dans le passage (19) de la partie de base (12), et une pièce de liaison intérieure sensiblement cylindrique creuse (33) à laquelle est raccordé un tuyau flexible (10) s'étendant dans le volume intérieur de la poche en film (2), et
la partie inférieure (20) de l'insert (13) et la partie supérieure (25) de l'insert sont d'un seul tenant.

2. Récipient selon la revendication 1,
**caractérisé en ce que** l'élément d'insertion extérieur sensiblement cylindrique creux (32) de la partie inférieure (25) de l'insert (13) est emboîté par encliquetage et/ou par serrage dans le passage (19) de la partie de base (12) de l'élément de raccordement côté poche (9).

3. Récipient selon la revendication 2,
**caractérisé en ce que** l'extrémité supérieure du tuyau flexible (10) est insérée dans la pièce de liaison intérieure sensiblement cylindrique creuse (33) de la partie inférieure (25) de l'insert (13).

4. Récipient selon l'une des revendications 1 à 3,
**caractérisé en ce que** la partie de base (12) comporte une bride périphérique supérieure (21).

5. Récipient selon l'une des revendications 1 à 4,
**caractérisé en ce que** l'insert (13) comporte un rebord périphérique (36) qui s'appuie sur la face supérieure de la bride périphérique (21) de la partie de base (12).

6. Récipient selon l'une des revendications 1 à 5,
**caractérisé en ce qu'**un capuchon de fermeture (15) est placé sur la partie supérieure (25) de l'insert (13).

7. Récipient selon l'une des revendications 1 à 6,
**caractérisé en ce que** le récipient (1) est rempli d'un concentré de dialyse liquide, en particulier d'un concentré acide, destiné à la préparation d'un liquide de dialyse prêt à l'emploi.

8. Récipient selon l'une des revendications 1 à 7,
**caractérisé en ce que** la partie de base (12) et l'insert (13) de l'élément de raccordement côté poche (9) sont des pièces moulées par injection réalisées par un procédé de moulage par injection.

9. Emballage comprenant un carton pliable (4) muni d'une partie de fond (50), de parties latérales (51, 52) et d'une partie de couvercle (49), ainsi qu'un récipient (1) selon l'une des revendications 1 à 8,
**caractérisé en ce que** le récipient (1) est inséré dans le carton pliable (4) de telle sorte que la poche en film (1) repose sur la partie de fond (50), la partie supérieure (25) de l'insert (13) s'étendant vers l'extérieur à travers la partie de couvercle (49).

10. Dispositif d'alimentation en concentré de dialyse destiné à fournir du concentré de dialyse pour la préparation d'un liquide de dialyse prêt à l'emploi dans un centre de dialyse ou un hôpital, le dispositif d'alimentation en concentré de dialyse (83) comportant au moins une conduite en tuyau flexible (61, 62) destinée à l'aspiration du concentré de dialyse, à l'extrémité libre de laquelle est prévu un élément de raccordement (37) destiné à établir une communication fluidique avec un élément de raccordement (9) d'un récipient (1) rempli de concentré de dialyse,
**caractérisé en ce que**
l'élément de raccordement (37) de la conduite en tuyau flexible (61, 62) est conçu à la manière d'une fiche femelle d'un raccord Camlock, qui est conçue pour recevoir une fiche conçue à la manière d'une fiche mâle (31) du raccord Camlock, et qui comporte deux bras de verrouillage (42) destinés à verrouiller la fiche mâle, la fiche femelle du raccord Camlock étant réalisée sous la forme d'un corps en forme de capuchon qui comporte une partie de base (38) dans laquelle est prévu un évidement (39), et une partie de couvercle (40), la partie couvercle (40) étant pourvue d'un embout de raccordement (41) auquel est raccordée l'extrémité libre de la conduite en tuyau flexible (61, 62).

11. Dispositif d'alimentation en concentré de dialyse selon la revendication 10, **caractérisé en ce que** l'axe longitudinal (82) de l'embout de raccordement (41) est incliné par rapport à l'axe longitudinal (48) de l'évidement (39).

12. Dispositif d'alimentation en concentré de dialyse selon la revendication 10 ou 11,
**caractérisé en ce que** les bras de verrouillage (42) sont fixés sur des côtés opposés de la partie de base (38) de manière à pouvoir pivoter dans un plan perpendiculaire au plan dans lequel se trouve l'axe longitudinal (82) de l'embout de raccordement (41), et/ou
**en ce que** la partie de base (38) comporte, sur des côtés opposés, des nervures latérales (43) qui délimitent une rainure (44), les bras de verrouillage (42) étant fixés sur les nervures latérales (43) de manière à pouvoir pivoter.

13. Dispositif d'alimentation en concentré de dialyse selon l'une des revendications 10 à 12,
**caractérisé en ce qu'**un joint d'étanchéité est disposé dans l'évidement (39) de la partie de base (38), lequel s'appuie contre la partie de couvercle (40) et présente une section transversale (47) sensiblement rectangulaire.

14. Procédé de fabrication d'un récipient destiné à être raccordé à une conduite en tuyau flexible d'un dispositif d'alimentation en concentré de dialyse afin de fournir du concentré de dialyse pour la préparation d'un liquide de dialyse prêt à l'emploi dans un centre de dialyse ou un hôpital, comprenant les étapes de procédé suivantes consistant à :
fournir une poche en film (2) constituée d'un film souple,
fournir un élément de raccordement côté poche (9), destiné à être relié à la poche en film, pour le raccordement d'un élément de raccordement côté tuyau (37) d'une conduite en tuyau flexible (61, 62) d'un dispositif d'alimentation en concentré de dialyse (83), l'élément de raccordement côté poche (9) comportant une partie de base (12) qui présente un passage (19),
fournir un insert (13) qui comporte un orifice (24) pour le concentré de dialyse, l'insert (13) comportant une partie supérieure (25) conçue comme une fiche mâle d'un raccord Camlock et dotée d'une rainure périphérique (27), et une partie inférieure (20) qui comprend un élément d'insertion extérieur sensiblement cylindrique creux (32) et une pièce de liaison intérieure sensiblement cylindrique creuse (33),
relier de manière étanche aux liquides la partie de base (12) de l'élément de raccordement côté poche (9) aux couches de film de la poche en film (2) sur la face supérieure (7) de la poche en film (2) en position d'utilisation,
fermer de manière étanche aux liquides la partie supérieure (25) de l'insert (13) à l'aide d'un élément de fermeture amovible ou détachable (14), en particulier un film pelable,
raccorder un tuyau flexible (10) à la pièce de liaison intérieure sensiblement cylindrique creuse (33) de la partie inférieure (20) de l'insert (13),
remplir la poche en film (2) avec le concentré de dialyse,
introduire le tuyau flexible (10) dans la poche en film (2) et insérer l'élément d'insertion extérieur sensiblement cylindrique creux (32) de la partie inférieure (20) de l'insert (13) dans le passage (19) de la partie de base (12) de l'élément de raccordement côté poche (9).

15. Procédé selon la revendication 14,
**caractérisé en ce que** la poche en film (2), avant d'être remplie, est insérée dans un carton pliable (4) muni d'une partie de fond (50), de parties latérales (51, 52) et d'une partie de couvercle (7), de telle sorte que la poche en film (2) repose sur la partie de fond (50) et que la partie supérieure (25) de l'insert (13) de l'élément de raccordement côté poche (9) s'étende vers l'extérieur à travers la partie de couvercle (7).
